# EUROPEAN PATENT SPECIFICATION

(11) **EP 0 589 044 B1**
(45) Date of publication and mention of the grant of the patent: **23.10.1996**
(21) Application number: 92924909.2
(22) Date of filing: 11.12.1992
(51) Int. Cl.: C07C 39/15, A01N 43/80, C07C 43/23, C07C 39/367

(54) **NOVEL INCLUSION COMPOUND COMPRISING TETRAKISPHENOL AS HOST**
NEUE EINSCHLUSSVERBINDUNGEN WELCHE TETRAKISPHENOL ALS WIRT BEINHALTEN
NOUVEAU COMPLEXE D'INSERTION COMPRENANT UN TETRAKISPHENOL COMME SUBSTANCE CAGE

(30) Priority: 12.12.1991 JP 350734/91; 19.12.1991 JP 353930/91; 19.05.1992 JP 150083/92; 17.07.1992 JP 213644/92; 01.12.1992 JP 345524/92
(43) Date of publication of application: 30.03.1994
(73) Proprietor: NIPPON SODA CO., LTD., Chiyoda-ku, Tokyo 100 (JP)
(72) Inventor: ASAI, Makoto R&D Lab. for Specialty Chemicals, Ichihara-shi Chiba 290 (JP); SUZUKI, Hiroshi R&D Lab. for Specialty Chemicals, Ichihara-shi Chiba 290 (JP); ICHIKAWA, Takako R&D Lab. for Specialty Chemicals, Ichihara-shi Chiba 290 (JP)
(74) Representative: de Bruijn, Leendert C.
(86) International application number: JP9201620
(87) International publication number: WO9312060

(56) References cited:
- JP-A- 1 192 711
- JP-A- 2 015 040
- JP-A- 2 036 219
- JP-A- 2 258 831
- JP-A- 3 200 254
- JP-A- 4 120 039
- JP-A-61 093 165
- JP-A-61 270 189
- JP-A-62 089 674
- JP-B-43 018 452
- TETRAHEDRON LETTERS vol. 33, no. 42 , 13 October 1992 , OXFORD GB pages 6319 - 6322 H SUZUKI ET AL 'INCLUSION COMPLEXES OF A NOVEL HOST 1,1,2,2-TETRAKIS(4-HYDROXYPHENY L)ETHANE,WITH VARIOUS GUESTS'
- DATABASE WPI Week 9337, Derwent Publications Ltd., London, GB; AN 93-290555 & JP-A-5 170 686 (NIPPON SODA CO)
- DATABASE WPI Week 9307, Derwent Publications Ltd., London, GB; AN 93-055205 & JP-A-5 004 978 (NIPPON SODA CO)
- DATABASE WPI, Week 9307, Derwent Publications Ltd., London, GB; AN 93-055205 & JP-A-5 004 978

## Description

This invention has an object to provide novel clathrate compounds which are based on tetrakisphenols as host and are useful in the technological fields of selective separation, chemical stabilization, conversion to non-volatile property, powder processing and the like.

This invention also has another object to solve several problems as described below regarding the conventional manufacturing process of clathrate compounds and to provide a simple and efficient process for manufacturing clathrate compounds. The problems to be solved by this invention are:
1) Clathrate compounds are not produced or guest molecules are not incorporated but instead clathrates including solvent are produced depending upon the kinds of solvents.
2) Even though the solvent is suitable to produce clathrate compounds, the conditions for the synthesis and the isolation of clathrate compounds are difficult since the conditions such as temperature, the ratio of host compound to guest compound, concentration and stirring are restricted in order to deposit the compounds including the guest molecule.
3) The recovery rate does not come to 100% on the basis of host compound.

### BACKGROUND OF THE INVENTION

A clathrate compound is a compound having a structure wherein a guest molecule occupies a space formed in a host molecule and it is recently expected to be applied for various technological fields such as selective separation, chemical stabilization, conversion to non-volatile property, powder processing and the like.

Clathrate compounds of 1,1,6,6-tetraphenyl-2,4-hexadiyne-1,6-diol or 1,1-bis(2,4-dimethylphenyl)-2-propyne-1-ol with 5-chloro-2-methyl-4-isothiazoline-3-one (CMI) are described in US-A-4,644,021 (JP-A-61-53201). Clathrate compounds of 1,1'-bis-2-naphthol with CMI are described in JP-A-62-A-22701. JP-A-61-93165 discloses the isolation of quinaldine with a benzpinacol-like compound having the formula [C₆H₅]₂C(OH)-(C≡C)ₙ-C(OH)[C₆H₅]₂ (n = 0-2). JP-A-62-A-89674 discloses complexes of a tetraphenyl compound having formula [XₙC₆H₅₋ₙ]₂C(OH)-C≡C-C≡C-C(OH)[C₆H₅₋ₙXₙ]₂ with glycidyl compounds; no complexes with substituted tetraphenyl compounds (n ≠ 0) are illustrated.

As a clathrate compound with tetrakisphenols as host, the one using tetrakis(4-hydroxyphenyl)ethane is described in Tetrahedron Letters, 33(42), 6319-6322 (1992), which was published in October 1992.

### DISCLOSURE OF THE INVENTION

The inventors of the present invention and the associates found that the novel clathrate compounds are produced very efficiently by directly adding the host compound comprising specific tetrakisphenols into the solution containing the guest compound to make reaction if the guest compound is liquid, by reacting it in the solution containing guest compound if the guest compound is solid, or by direct solid-phase reaction with solid guest compound, thereby completing this invention.

The details of the present invention are illustrated hereinbelow.

### (Host compounds)

The host compound comprising tetrakisphenol used in the present invention is a compound represented by the general formula [I]. 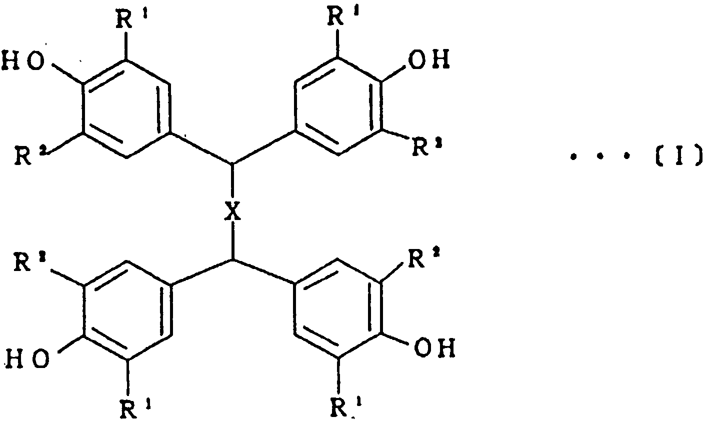 wherein X represents (CH₂)n, n is 0, 1, 2 or 3, R¹ and R² represent each independently hydrogen, C₁₋₄ alkyl, unsubstituted or substituted phenyl, halogen or C₁₋₄ alkoxy.

R¹ and R² in the general formula [I] are identical or different and are, for example, hydrogen, C₁₋₄ alkyl such as methyl, ethyl, propyl, isopropyl, n-butyl, isobutyl and tert-butyl, phenyl halogen such as fluorine, chlorine, bromine and iodine, or C₁₋₄ alkoxy such as methoxy, ethoxy and tert-butoxy.

The tetrakisphenols used in the present invention are not particularly restricted as far as they are compounds represented by the general formula [I]. The following compounds are included in the tetrakisphenols as examples.

1,1,2,2-Tetrakis(4-hydroxyphenyl)ethane, 1,1,2,2-tetrakis(3-methyl-4-hydroxyphenyl)ethane, 1,1,2,2-tetrakis(3,5-dimethyl-4-hydroxyphenyl) ethane, 1,1,2,2-tetrakis(3-chloro-4-hydroxyphenyl)ethane, 1,1,2,2-tetrakis(3,5-dichloro-4-hydroxyphenyl)ethane, 1,1,2,2-tetrakis(3-bromo-4-hydroxyphenyl)ethane, 1,1,2,2-(3,5-dibromo-4-hydroxyphenyl)ethane, 1,1,2,2-tetrakis(3-tert-butyl-4-hydroxyphenyl)ethane, 1,1,2,2-tetrakis(3, 5-ditert-butyl-4-hydroxyphenyl)ethane, 1,1,2,2-tetrakis(3-fluoro-4-hydroxyphenyl)ethane, 1,1,2,2-tetrakis(3,5-difluoro-4-hydroxyphenyl) ethane, 1,1,2,2-tetrakis(3-methoxy-4-hydroxyphenyl)ethane, 1,1,2,2-tetrakis(3,5-dimethoxy-4-hydroxyphenyl)ethane, 1,1,2,2-tetrakis(3-chloro-5-methyl-4-hydroxyphenyl)ethane, 1,1,2,2-tetrakis(3-bromo-5-methyl-4-hydroxyphenyl)ethane, 1,1,2,2-tetrakis(3-methoxy-5-methyl-4-hydroxyphenyl)ethane, 1,1,2,2-tetrakis(3-tert-butyl-5-methyl-4-hydroxyphenyl)ethane, 1,1,2,2-tetrakis(3-chloro-5-bromo-4-hydroxyphenyl) ethane, 1,1,2,2-tetrakis(3-chloro-5-phenyl-4-hydroxyphenyl)ethane, 1,1,2, 2-tetrakis[(4-hydroxy-3-phenyl)phenyl]ethane, 1,1,3,3-tetrakis(4-hydroxyphenyl)propane, 1,1,3,3-tetrakis(3-methyl-4-hydroxyphenyl)propane, 1,1,3,3-tetrakis(3,5-dimethyl-4-hydroxyphenyl)propane, 1,1,3,3-tetrakis(3-chloro-4-hydroxyphenyl)propane, 1,1,3,3-tetrakis(3,5-dichloro-4-hydroxyphenyl)propane, 1,1,3,3-tetrakis(3-bromo-4-hydroxyphenyl)propane, 1,1,3,3-tetrakis(3,5-dibromo-4-hydroxyphenyl) propane, 1,1,3,3-tetrakis(3-phenyl-4-hydroxyphenyl)propane, 1,1,3,3-tetrakis(3,5-diphenyl-4-hydroxyphenyl)propane, 1,1,3,3-tetrakis(3-methoxy-4-hydroxyphenyl)propane, 1,1,3,3-tetrakis(3,5-dimethoxy-4-hydroxyphenyl)propane, 1,1,3,3-tetrakis(3-tert-butyl-4-hydroxyphenyl) propane, 1,1,4,4-tetrakis(3,5-ditert-butyl-4-hydroxyphenyl)butane, 1,1,5,5-tetrakis(4-hydroxyphenyl)pentane, 1,1,5,5-tetrakis(3-methyl-4-hydroxyphenyl)pentane, 1,1,5,5-tetrakis(3,5-dimethyl-4-hydroxyphenyl) pentane, 1,1,5,5-tetrakis(3-chloro-4-hydroxyphenyl)pentane, 1,1,5,5-tetrakis(3,5-dichloro-4-hydroxyphenyl)pentane, 1,1,5,5-tetrakis(3-bromo-4-hydroxyphenyl)pentane, 1,1,5,5-tetrakis(3,5-dibromo-4-hydroxyphenyl)pentane, 1,1,5,5-tetrakis(3-methoxy-4-hydroxyphenyl) pentane, 1,1,5,5-tetrakis(3,5-dimethoxy-4-hydroxyphenyl)pentane, 1,1,5,5-tetrakis(3-tert-butyl-4-hydroxyphenyl)pentane, 1,1,5,5-tetrakis(3,5-ditert-butyl-4-hydroxyphenyl)pentane, 1,1,5,5-tetrakis(3-methyl-5-tert-butyl-4-hydroxyphenyl)pentane, 1,1,5,5-tetrakis(3-phenyl-4-hydroxyphenyl)pentane, 1,1,5,5-tetrakis(3,5-diphenyl-4-hydroxyphenyl) pentane, 1,1,5,5-tetrakis(3,5-dicyclohexyl-4-hydroxyphenyl)pentane, 1,1, 4,4-tetrakis(4-hydroxyphenyl)butane, 1,1,4,4-tetrakis(3-methyl-4-hydroxyphenyl)butane, 1,1,4,4-tetrakis(3,5-dimethyl-4-hydroxyphenyl) butane, 1,1,4,4-tetrakis(3-chloro-4-hydroxyphenyl)butane, 1,1,4,4-tetrakis(3,5-dlchloro-4-hydroxyphenyl)butane, 1,1,4,4-tetrakis(3-methoxy-4-hydroxyphenyl)butane, 1,1,4,4-tetrakis(3,5-dimethoxy-4-hydroxyphenyl)butane, 1,1,4,4-tetrakis(3-bromo-4-hydroxyphenyl)butane and 1,1,4,4-tetrakis(3,5-dibromo-4-hydroxyphenyl)butane.

### (Guest compounds)

The organic compounds capable of forming clathrate compounds of which the host compound comprises a tetrakisphenol having formula I wherein n ≠ 0 and/or R¹ ≠ H, can be used as the guest organic compound of the present invention without particular restriction. Examples of the above guest organic compounds are alcohols such as methanol, ethanol, i-propanol, n-butanol, 2-ethylhexanol and the like; aldehydes such as formaldehyde, acetaldehyde, benzaldehyde and the like; ketones such as acetone, methylethylketone and the like; nitriles such as acetonitrile and the like; ethers such as tetrahydrofuran, diethylether and the like; esters such as methyl acetate, ethyl acetate, butyl acetate and the like. Organic compounds that can be used with any tetrakisphenol having formula I include natural essential oils such as 1,8-cineole, hinokithiol, menthol, terpineol, borneol, nopol, citral, citronellol, citronellal, geraniol, linalool, dimethyloctanol and the like; synthetic perfumes such as fragrant orange-colored olive, jasmine, lemon and the like; monocyclic nitrogen-containing heterocyclic compounds such as pyridine, pyrrole, imidazole, pyrazine, pyrazole, 1,2,4-triazole, thiazole, pyrrolidine, imidazoline, pyrroline, oxazole, piperine, pyrimidine, pyridazine, triazine, 5-chloro-2-methyl-4-isothiazoline-3-one and the like.

The compound of the present invention can be obtained as novel clathrate compound by reacting the said guest organic compound or the solution containing the guest organic compound with tetrakisphenol being host compound with stirring for several minutes to several hours in the temperature range from room temperature to 100 °C to include the guest organic compound into the host compound easily.

The said clathrate compound can be utilized for the separation and the recovery of guest compounds from the solution in water based on the characteristics that the clathrate compounds easily release the guest compound when heated under reduced pressure and the host compound does not include water.

According to the present invention, when the guest compound is liquid, the clathrate compound can be produced with high selectivity and high yields by directly reacting the guest compound with powdered host compound.

On the other hand, when the guest compound is solid, the clathrate compound is also produced with high selectivily and high yields by reacting the solution or the solid of the guest compound with the powdered host compound.

The clathrate compounds of the present invention result from the invasion of the molecules of the guest compound into a hollow formed by the host compound molecules. Therefore, the degree that the compound is taken into the host compound as the guest compound defends on the size, the steric structure, the polarity and the solubility of the molecules of the guest compound.

### BRIEF DESCRIPTION OF THE DRAWINGS

FIG. 1 is an infrared spectrum of host compound, 1,1,2,2-tetrakis(4-hydroxyphenyl)ethane (hereinafter abbreviated to TEP-DF).
FIG. 2 is a X-ray diffraction diagram of TEP-DF.
FIG. 3 is an infrared spectrum of the sample A-1.
FIG. 4 is an infrared spectrum of the sample A-2.
FIG. 5 is a X-ray diffraction diagram of the sample of A-1.
FIG. 6 is a X-ray diffraction diagram of the sample of A-2.
FIG. 7 is a TG/DTA (Thermogravimetry-Differential thermal analysis) chart of the sample A-1.
FIG. 8 is a TG/DTA chart of the sample A-2.
FIG. 9 is a TG/DTA chart of TEP-DF.
FIG. 10 is a DTA chart of the sample A-11.
FIG. 11 is an infrared spectrum of the sample A-11.
FIG. 12 is an infrared spectrum of the guest compound, hinokithiol.
FIG. 13 is a X-ray diffraction diagram of the sample of A-11.
FIG. 14 is a X-ray diffraction diagram of the guest compound, hinokithiol.
FIG. 15 shows respective release curve of A-10 and 1,8-cineole obtained from the release amount measured at the release tests.
FIG 16 shows respective release curve of each of A-11 and hinokithiol obtained from the release amount measured at the release tests.
FIG. 17 is an infrared spectrum (KBr) of 1,1,2,2-tetrakis(3,5-dimethyl-4-hydroxyphenyl)ethane obtained in the synthetic Example 1-1.
FIG. 18 is a ¹H-NMR spectrum (d⁰-Acetone, TMS) of 1,1,2,2-tetrakis(3,5-dimethyl-4-hydroxyphenyl)ethane obtained in the synthetic Example 1-1.
FIG. 19 is an infrared spectrum (KBr) of 1,1,2,2-tetrakis(3-methyl-4-hydroxyphenyl)ethane obtained in the synthetic Example 1-2.
FIG. 20 is a ¹H-NMR spectrum (d-MeOH, TMS) of 1,1,2,2-tetrakis(3-methyl-4-hydroxyphenyl)ethane obtained in the synthetic Example 1-2.
FIG. 21 is an infrared spectrum (KBr) of 1,1,2,2-tetrakis(3-chloro-4-hydroxyphenyl)ethane obtained in the synthetic Example 1-3.
FIG. 22 is a ¹H-NMR spectrum (d-MeOH, TMS) of 1,1,2,2-tetrakis(3-chloro-4-hydroxyphenyl)ethane obtained in the synthetic Example 1-3.
FIG. 23 is an infrared spectrum (KBr) of 1,1,2,2-tetrakis(3-bromo-4-hydroxyphenyl)ethane obtained in the synthetic Example 1-4.
FIG. 24 is a ¹H-NMR spectrum (d-MeOH, TMS) of 1,1,2,2-tetrakis(3-bromo-4-hydroxyphenyl)ethane obtained in the synthetic Example 1-4.
FIG. 25 is an infrared spectrum (KBr) of 1,1,4,4-tetrakis(4-hydroxyphenyl)butane obtained in the synthetic Example 1-6.
FIG. 26 is a ¹H-NMR spectrum (d-MeOH, TMS) of 1,1,4,4-tetrakis(4-hydroxyphenyl)butane obtained in the synthetic Example 1-6.
FIG. 27 is an infrared spectrum (KBr) of 1,1,5,5-tetrakis(4-hydroxyphenyl)pentane obtained in the synthetic Example 1-5.
FIG. 28 is a ¹H-NMR spectrum (d-MeOH, TMS) of 1,1,5,5-tetrakis(4-hydroxyphenyl)pentane obtained in the synthetic Example 1-5.
FIG 29 is an infrared spectrum (KBr) of the clathrate compound (A-21) wherein the host compound is 1,1,2,2-tetrakis(3-methyl-4-hydroxyphenyl)ethane obtained in synthetic Example 1-2 and the guest compound is acetone.
FIG. 30 is an infrared spectrum (KBr) of the clathrate compound (A-28) wherein the host compound is 1,1,2,2-tetrakis(3-chloro-4-hydroxyphenyl)ethane obtained in synthetic Example 1-3 and the guest compound is acetonitrile.
FIG 31 is an infrared spectrum (KBr) of the clathrate compound (A-36) wherein the host compound is 1,1,5,5-tetrakis(4-hydroxyphenyl)pentane obtained in synthetic Example 1-5 and the guest compound is 1,4-dioxane.
FIG. 32 is an infrared spectrum (KBr) of the clathrate compound (A-38) wherein the host compound is 1,1,4,4-tetrakis(4-hydroxyphenyl)butane obtained in synthetic Example 1-6 and the guest compound is 1,4-dioxane.
FIG. 33 is an infrared spectrum (KBr) of the clathrate compound (A-39) wherein the host compound is 1,1,2,2-tetrakis(3-methyl-4-hydroxyphenyl)ethane obtained in synthetic Example 1-1 and the guest compound is 1,8-cineole.
FIG. 34 is an infrared spectrum (KBr) of the clathrate compound (A-41) wherein the host compound is 1,1,2,2-tetrakis(3-methyl-4-hydroxyphenyl)ethane obtained in synthetic Example 1-2 and the guest compound is thymol.
FIG. 35 is a diagram of an infrared spectrum of the sample of clathrate compound A-52.
FIG. 36 shows a diagram of an infrared spectrum of CMI isolated from the guest compound, water soluble disinfectant (Kathon WT).
FIG. 37 shows a X-ray diffraction diagram of the clathrate compound sample A-52.
FIG. 38 shows a X-ray diffraction diagram of CMI isolated from the guest compound, water soluble disinfectant (Kathon WT).
FIG. 39 shows respective release curve of obtained sample A-52, sample for comparison C-1 and CMI obtained from the release amount measured at the release tests.

### BEST MODE FOR CARRYING OUT THE INVENTION

The present invention is illustrated in more detail by the following Examples. However, the scope of the present invention should not be limited by those Examples.

All samples A-1 through A-49 obtained in the Examples were confirmed as those are desired clathrate compounds by the analytical means such as infrared spectrum, NMR spectrum, DTA, X-ray diffraction and the like.

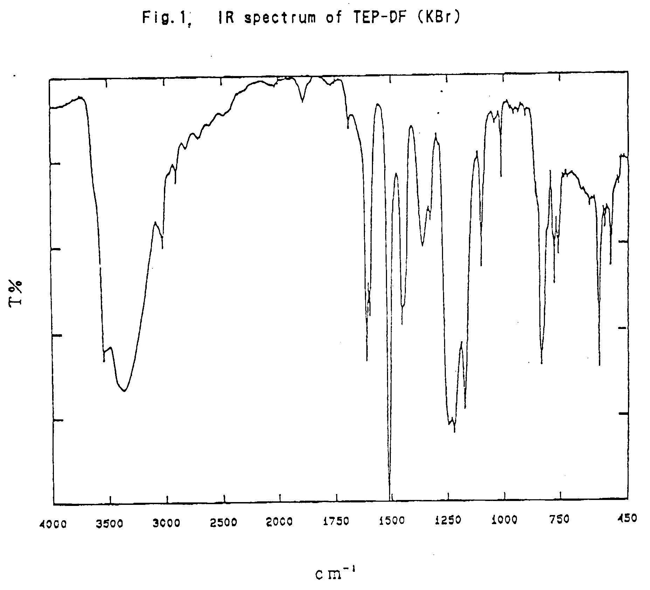

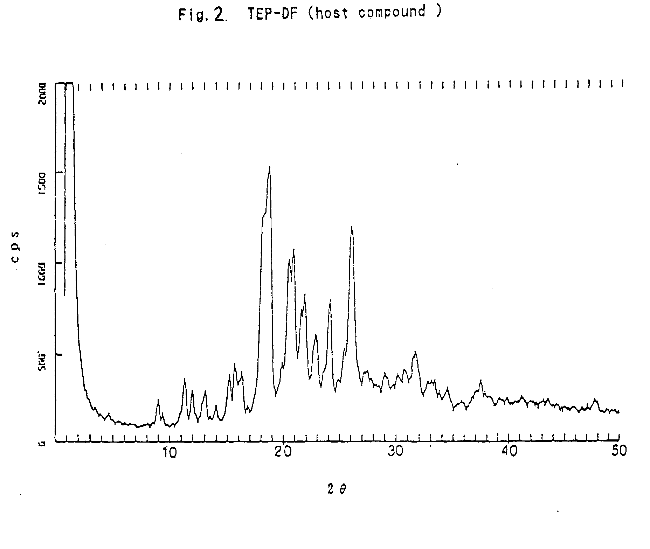

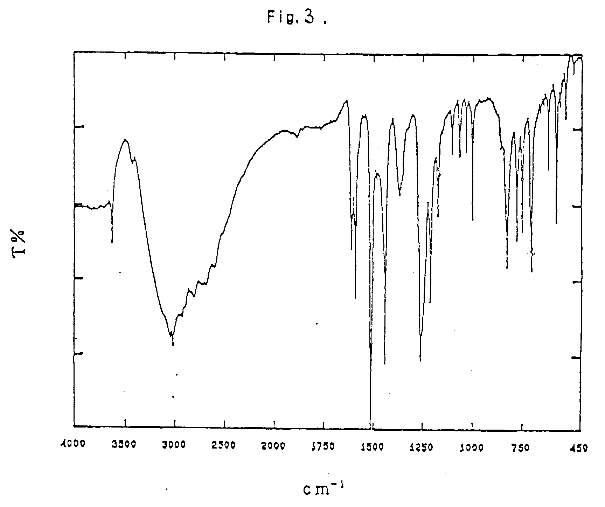

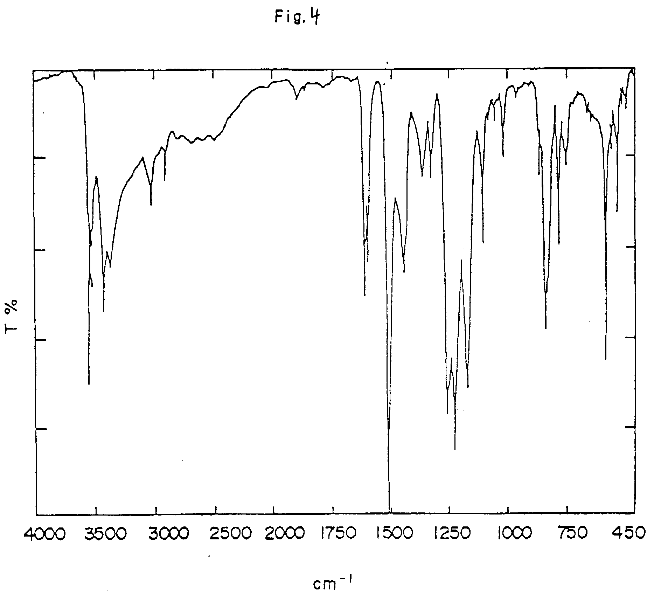

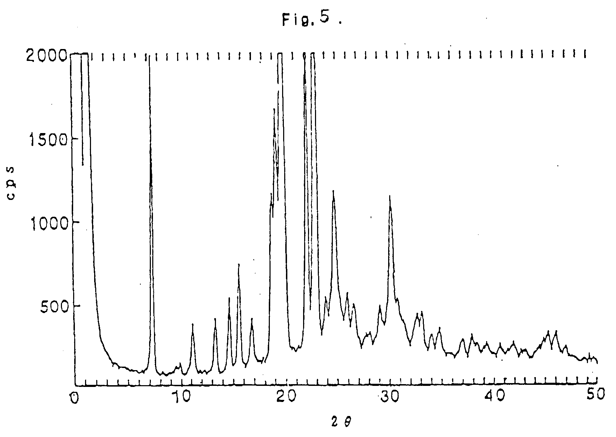

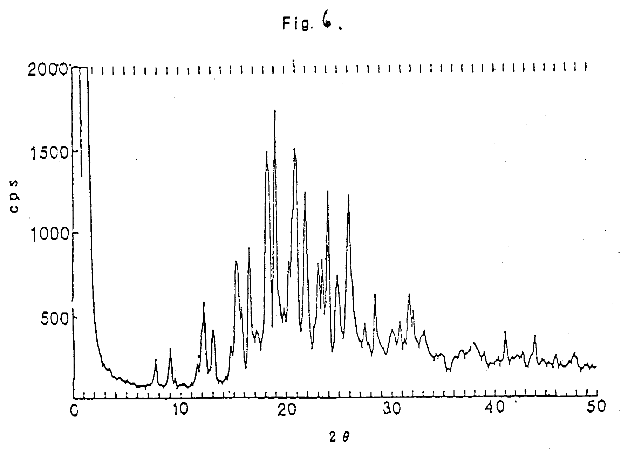

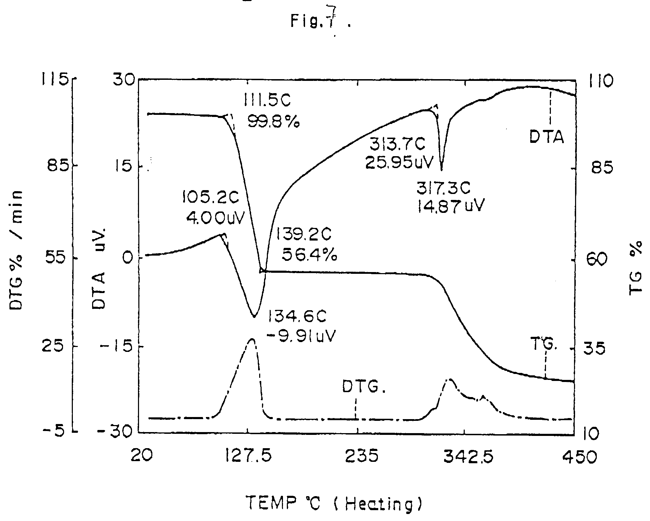

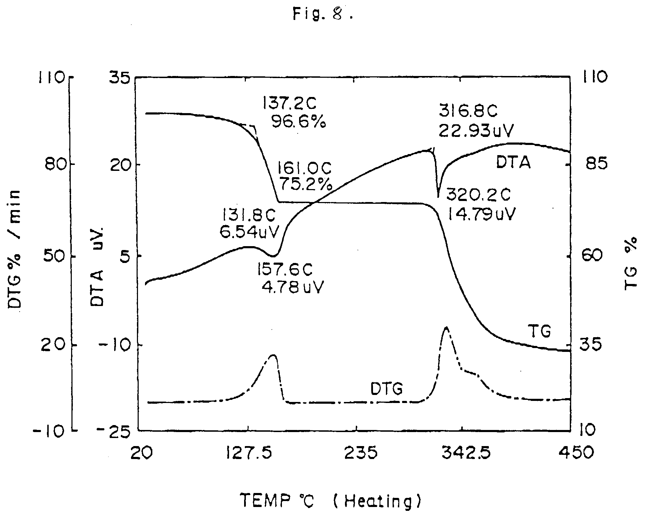

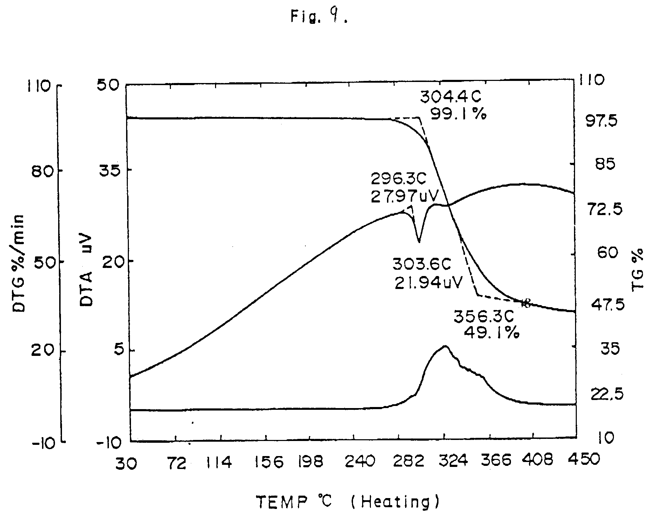

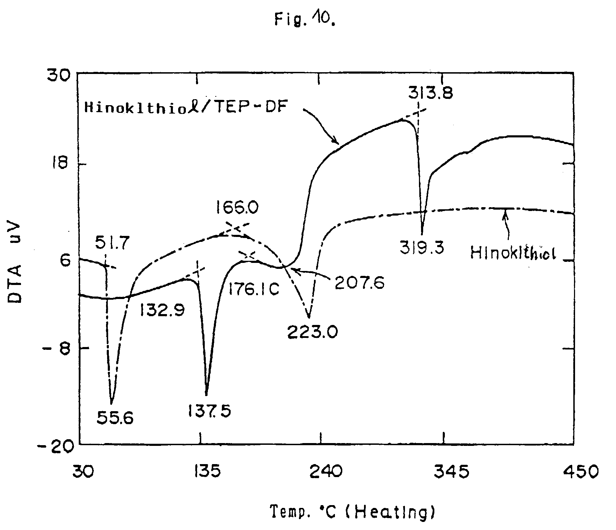

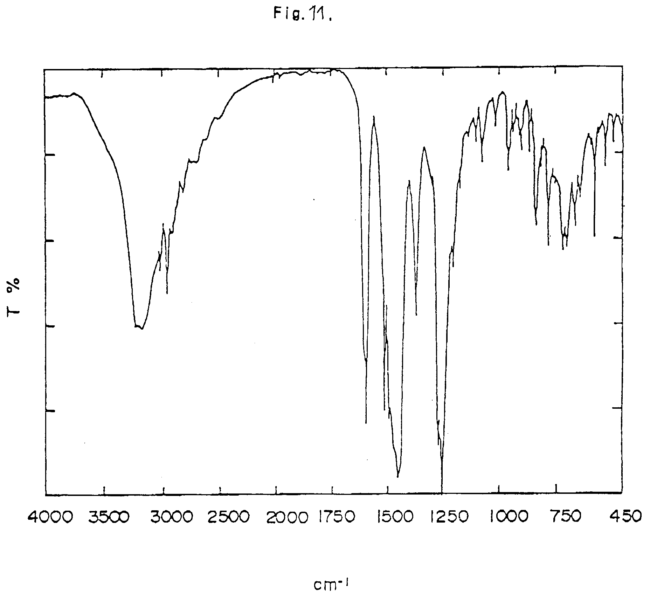

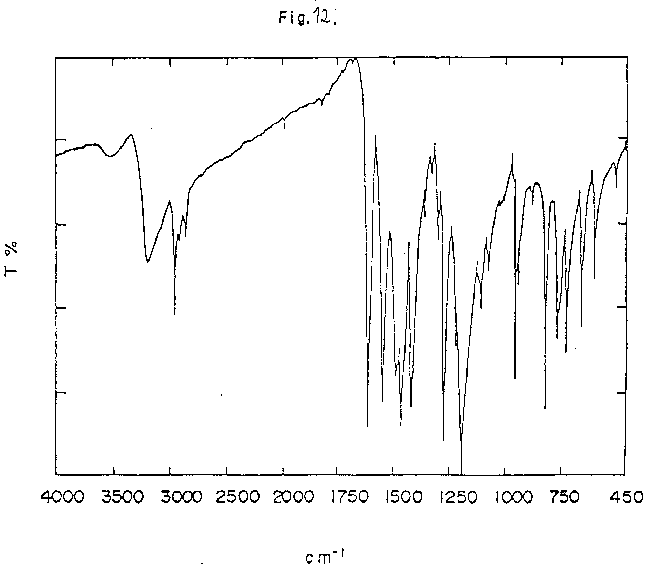

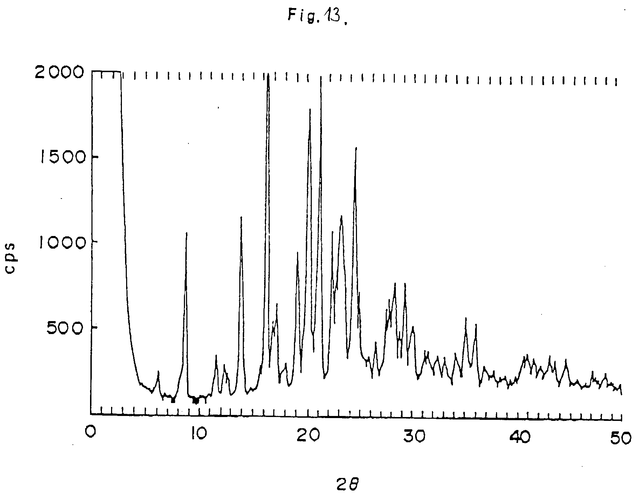

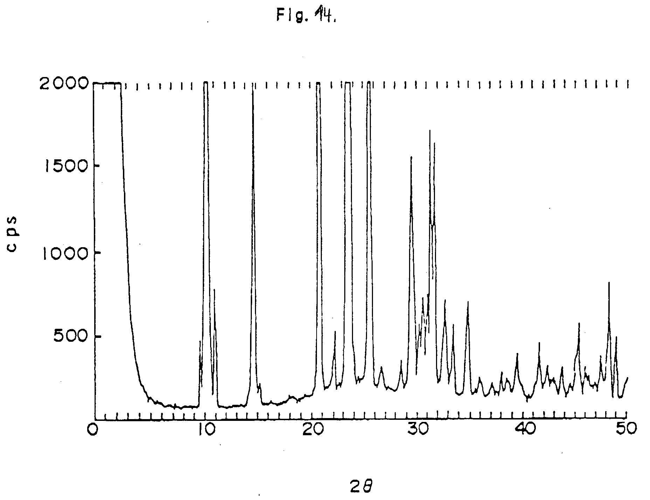

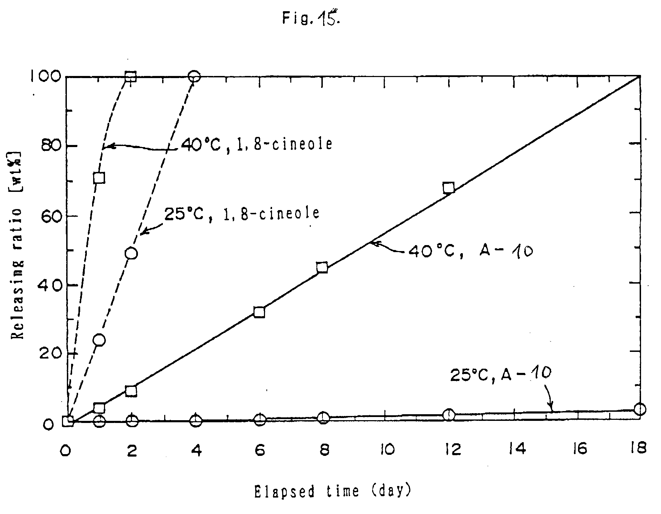

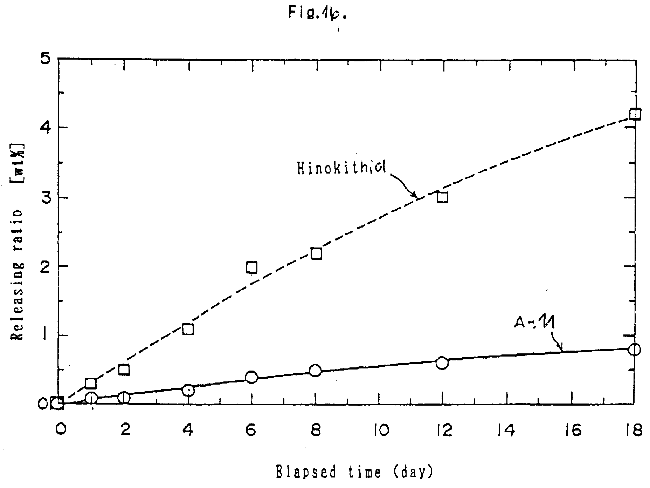

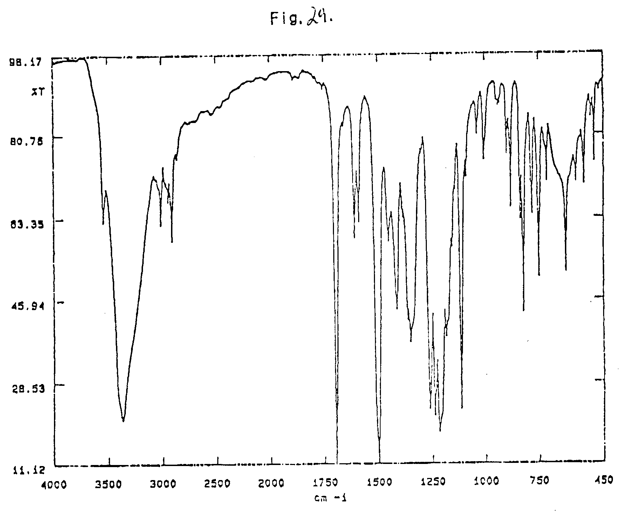

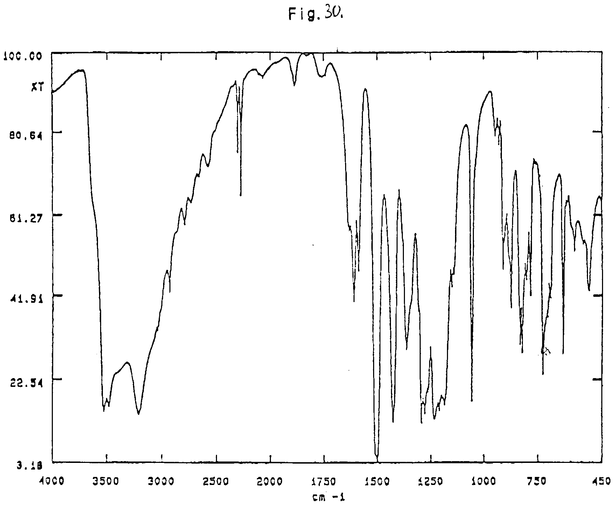

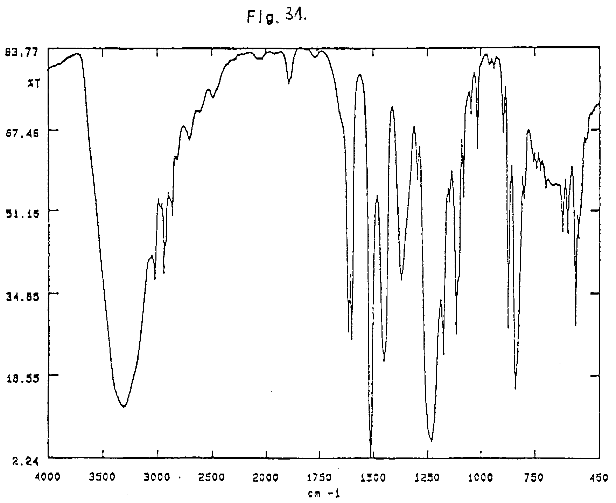

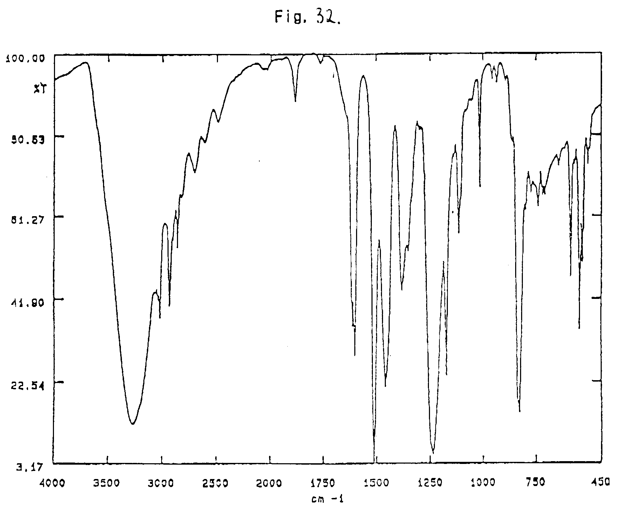

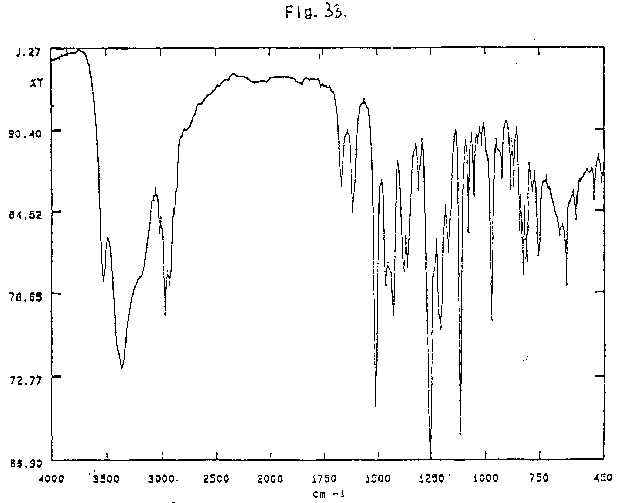

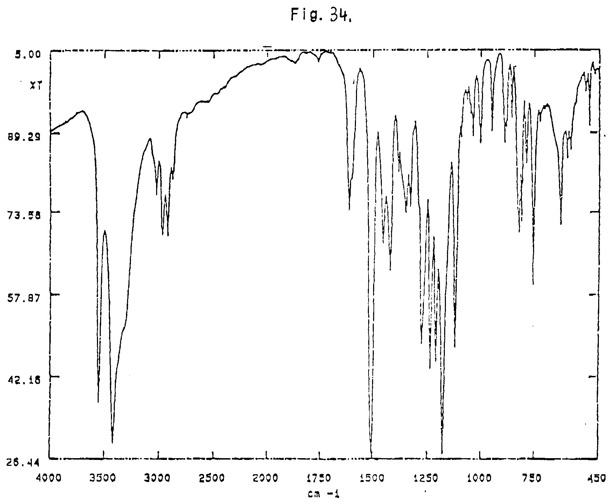

The chemical analytical data of the representative clathrate compounds obtained in the Examples and host compounds and guest compounds those are the materials for clathrate compounds are illustrated in FIG. 1 through 39.

### Example 1

### Sample: Preparation of A-1 and A-2

1.0 g (2.51 mmol) of TEP-DF (Asahi Yukizai Industries) was added into 5 ml of methanol and the mixture was stirred with heating until TEP-DF completely dissolved in the solution. Then 5 ml of pyridine (or pyrrole) which is a corresponding guest confound was gradually fed dropwise into the solution and stirred for 10 minutes at 60 °C. Then the reaction mixture was immediately filtered and the filtrate was allowed to stand at room temperature to deposit the crystals. The crystals were collected by filtration and dried under vacuum at room temperature, affording the sample in whitish powder of the compound specified in the present invention; A-1 and A-2. The yield was 84 % for A-1 and 70 % for A-2 in conversion to the host compound.

### Example 2

### Sample: Preparation of A-3

1.0 g (2.51 mmol) of TEP-DF was added into 5 ml of isopropanol and the mixture was stirred with heating until TEP-DF completely dissolved in the solution. Then 5 ml of pyrrole was gradually fed dropwise into the solution and stirred for 30 minutes at 80°C. Then the reaction mixture was immediately filtered and the filtrate was allowed to stand at room temperature to deposit the crystals. The crystals were collected by filtration and dried under vacuum at room temperature, affording the sample in whitish powder of the compound specified in the present invention: A-3. The yield was 68 % when calculated with hose compound.

### Example 3

### Sample: Preparation of A-4 through A-7

1.0 g (2.51 mmol) of TEP-DF was added into 5 ml of methanol and the mixture was stirred with heating until TEP-DF completely dissolved in the solution. Then 10.04 ml of corresponding guest compound (pyrazine, pyrazole, imidazole, 1,2,4-triazole) was added into the solution and stirred for 20 minutes at 60°C. Then the reaction mixture was immediately filtered and the filtrate was allowed to stand at room temperature to precipitate the crystals. The crystals were collected by filtration and dried under vacuum at room temperature, affording the sample in whitish powder of the compound specified in the present invention: A-4 through A-7. The yields were 82 % for A-4, 85 % for A-5, 73 % for A-6 and 95 % for A-7 respectively in conversion to the host compound.

### Example 4

### Sample: Preparation of A-8 and A-9

1.0 g (2.51 mmol) of TEP-DF was added into 5 ml of guest compound, pyridine (or pyrrole) and the gel suspension was obtained after stirring at room temperature. After separating the suspended materials in the suspension by filtration, the filtrate was dried under vacuum at room temperature, affording the sample in whitish powder of the compound specified in the present invention: A-8 and A-9. The yields were 100 % for A-8 and 100 % for A-9 in conversion to the host compound. The manufacturing condition of respective sample, guest/host mol ratio of the clathrate compounds obtained and the releasing temperature of the guest compounds were summarized in Table 1.

### Example 5

### Sample: Preparation of A-10

1.0 g (2.51 mmol) of TEP-DF (Asahi Yukizai Industries) was added into 5 ml of methanol and the mixture was stirred with heating until TEP-DF completely dissolved in the solution. Then 1.55 g (10.04 mmol) of 1,8-cineole was gradually fed dropwise into the solution and stirred for 10 minutes at 60°C to react the solution. Then the reactant solution was immediately filtered and the filtrate was allowed to stand at room temperature to deposit the crystals. The crystals were collected by filtration and dried under vacuum at room temperature, affording the sample in whitish powder A-10.

### Example 6

### Sample: Preparation of A-11

1.0 g (2.51 mmol) of TEP-DF was added into 5 ml of methanol and the mixture was stirred with heating until TEP-DF completely dissolved in the solution. Then 1.65 g (10.04 mmol) of hinokithiol was gradually fed dropwise into the solution and stirred for 10 minutes at 60 °C. Then the reaction mixture was immediately filtered and the filtrate was allowed to stand at room temperature to deposit the crystals. The crystals were collected by filtration and dried under vacuum at room temperature, affording the sample in whitish powder A-11.

### Example 7

### Sample: Preparation of A-12

1.0 g (2.51 mmol) of TEP-DF was added into 5 ml of benzene and dispersed in suspension by stirring. Then 1.57 g (10.04 mmol) of 1-menthol was added into the suspension, stirred for 30 minutes at 40°C and then cooled to room temperature. The suspension was separated by filtration and the filtrate was dried under vacuum at room temperature, affording the sample in whitish powder A-12.

### Example 8

### Sample: Preparation of A-13

1.0 g (2.51 mmol) of TEP-DF was added into 1.55 g (10. 04 mmol) of α-terpineol and the mixture was reacted at 60°C under stirring, then a solid substance was obtained. After grinding this solid substance, the resulting powder was dried under vacuum, affording the sample in whitish powder A-13.

The manufacturing condition of respective samples obtained in the above, guest/host mol ratio in the clathrate compounds obtained and the releasing temperature of the guest compounds were summarized in Table 2.

### Example 9

The obtained samples, A-10 and A-11 and comparative samples, 1,8-cineole (R-1) and hinokithiol (R-2) equivalent containing 0.6 g of guest compound are taken into Petri dish respectively and set in the desiccators maintained at the temperature of 25 and 40°C. Dried air was introduced into the desiccator at rate of 250 ml/min and the decrease of the weight was measured periodically. The results were shown in Table 3.

### (1) Synthesis of host compound (Synthetic Example 1)

### (1-1) Synthesis of 1,1,2,2-tetrakis(3,5-dimethyl-4-hydroxyphenyl)ethane:

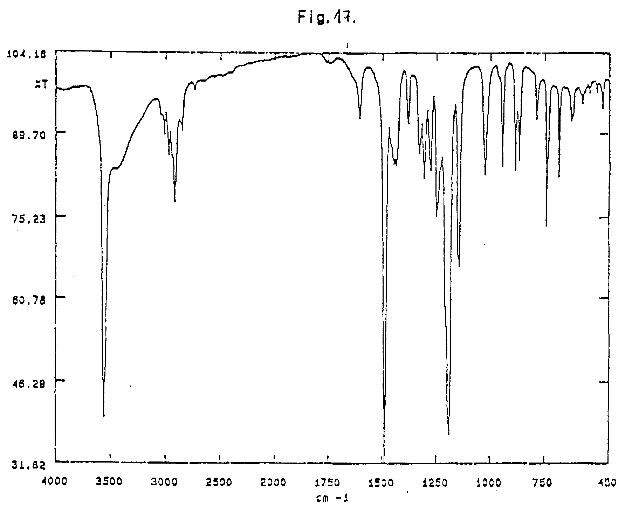

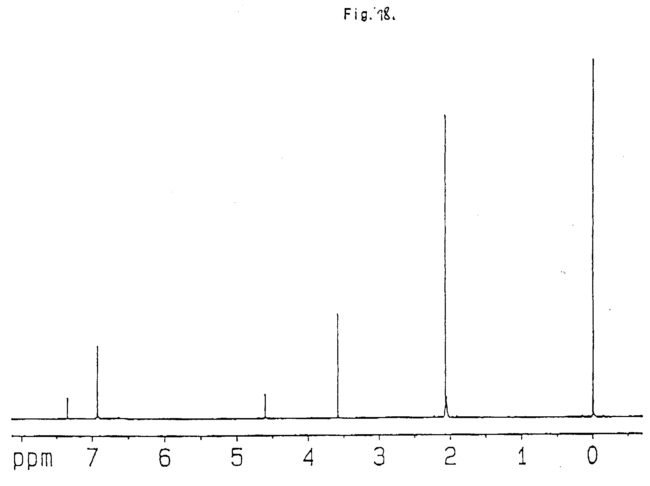

14.5 g of 40 % aqueous solution of glyoxal and 54.9 g of 2,6-xylenol were applied into 300 ml flask, then 100 ml of the mixture of concentrated sulfuric acid and phosphoric acid (ratio 3:1, v/v) was fed dropwise with stirring over 2 hours at the titration temperature of 0-2 °C. After titration, the mixture was stirred for 4 hours at 0°C. The reation mixture was poured into 600 ml of ice-water, then the temperature of the solution was raised to 70 °C to collect the deposited solid by filtration. The solid was recrystallized from dioxane, then dried under vacuum at 100 °C, affording 15.0 g of whitish powder. The yield was 29.4 %. The powder was identified as 1,1,2,2-tetrakis(3,5-dimethyl-4-hydroxyphenyl)ethane by means of both infrared spectrum and NMR spectrum. The chart of each measurement was shown in FIG. 17 and FIG. 18.

### (1-2) Synthesis of 1,1,2,2-tetrakis(3-methyl-4-hydroxyphenyl)ethane:

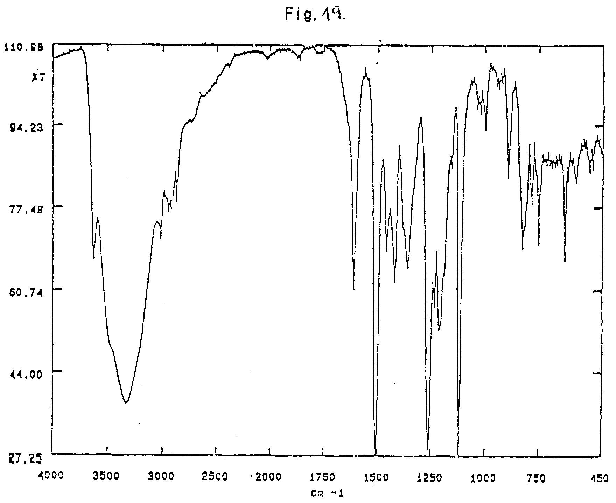

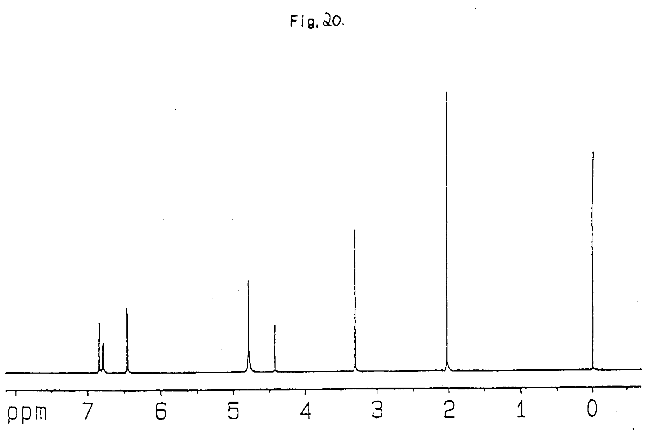

14.5 g of 40 % aqueous solution of glyoxal and 51.8 g of o-cresol were applied into 300 ml flask, then 100 ml of the mixture of concentrated sulfuric acid and phosphoric acid (volumetric ratio 3:1) was fed dropwise with stirring over 2 hours at the titration temperature of 0-2 °C. The mixture was stirred for 4 hours at 0 °C. After the end of the reaction, the reaction mixture was poured into 600 ml of ice-water, then the temperature of the solution was raised to 70 °C to collect the deposited solid by filtration. The solid was recrystallized form dioxane, then dried under vacuum at 100°C, affording 17.0 g of whitish powder. The yield was 37.4 %. The powder was identified as 1,1,2, 2-tetrakis(3-methyl-4-hydroxyphenyl)ethane by means of both infrared spectrum and NMR spectrum. The chart of each measurement was shown in FIG. 19 and FIG. 20.

### (1-3) Synthesis of 1,1,2,2-tetrakis(3-chloro-4-hydroxyphenyl)ethane:

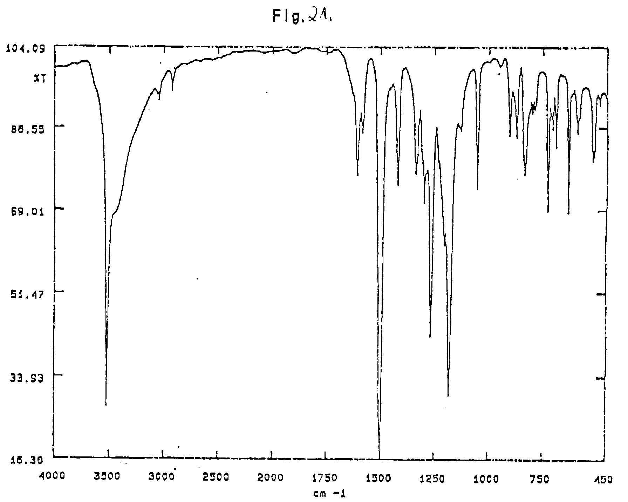

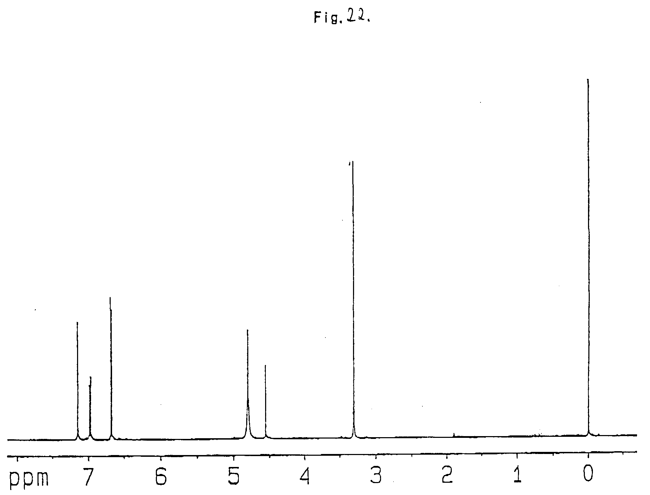

14.5 g of 40 % aqueous solution of glyoxal and 56.6 g of 2-chlorophenol were applied into 300 ml flask, then 100 ml of the mixture of concentrated sulfuric acid and phosphoric acid (volumetric ratio 3:1) was fed dropwise with stirring over 2 hours at the titration temperature of 0-2 °C. Following that, the mixture was stirred for 4 hours at 0 °C. The reaction mixture was poured into 600 ml of ice-water, then it was extracted three times with 100 ml of ether after the addition of NaCl. The ether layer was added with sodium sulfate anhydride and dehydrated, then ether and unreacted 2-chlorophenol were removed by distillation under reduced pressure. After the distillation, the residues were recrystallized from dioxane, then dried under vacuum at 100°C, affording 7.5 g of whitish powder. The yield was 14.0 %. The powder was identified as 1,1,2,2-tetrakis(3-chloro-4-hydroxyphenyl) ethane by means of both infrared spectrum and NMR spectrum. The chart of each measurement was shown in FIG. 21 and FIG. 22.

### (1-4) Synthesis of 1,1,2,2-tetrakis(3-bromo-4-hydroxyphenyl)ethane:

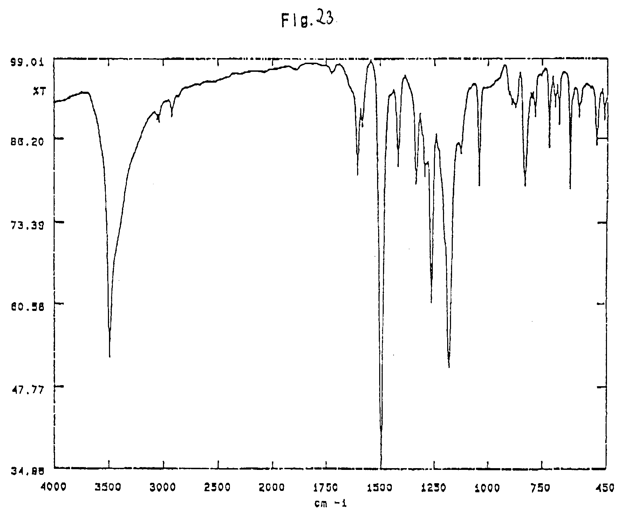

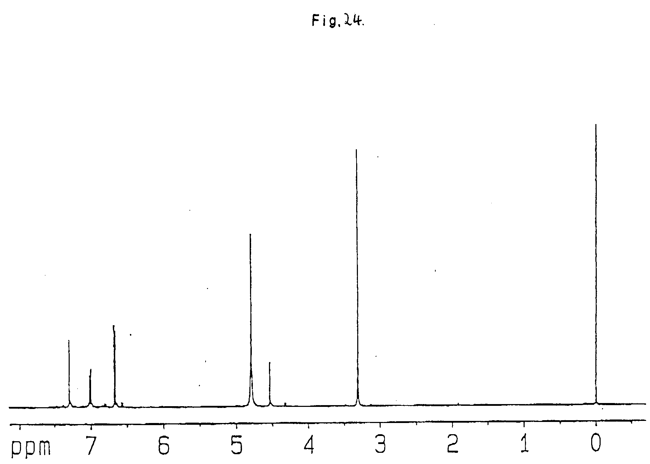

10.9 g of 40 % aqueous solution of glyoxal and 57.1 g of 2-bromophenol were applied into 300 ml flask, then 70 ml of the mixture of concentrated sulfuric acid and phosphoric acid (volumetric ratio 3:1) was fed dropwise with stirring over 1.5 hours at the titration temperature of 0-2°C. Following that, the mixture was stirred for 5 hours at 0 °C. The reaction mixture was poured into 500 ml of ice-water, then it was extracted three times with 100 ml of ether after the addition of NaCl. The ether layer was added with sodium sulfate anhydride and dehydrated, then ether and unreacted 2-bromophenol were removed by distillation under reduced pressure. After the distillation, the residues were recrystallized from dioxane, then dried under vacuum at 100 °C, affording 3.1 g of whitish powder. The yield was 5.8 %. The powder was identified as 1,1,2,2-tetrakis (3-bromo-4-hydroxyphenyl) ethane by means of both infrared spectrum and NMR spectrum. The chart of each measurement was shown in FIG. 23 and FIG. 24.

### (1-5) Synthesis of 1,1,5,5-tetrakis(4-hydroxyphenyl)pentane:

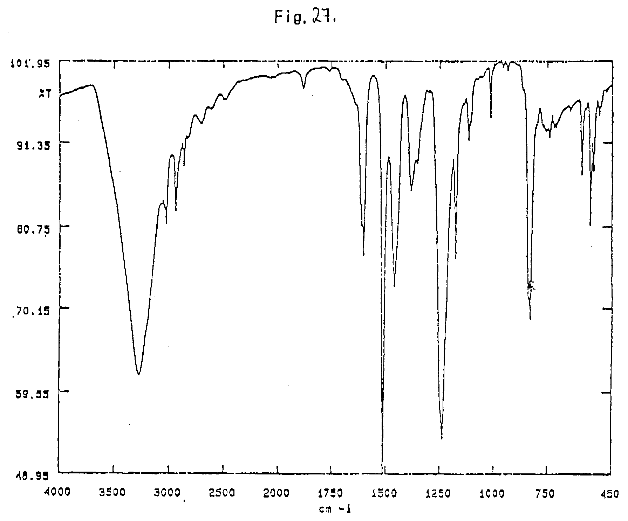

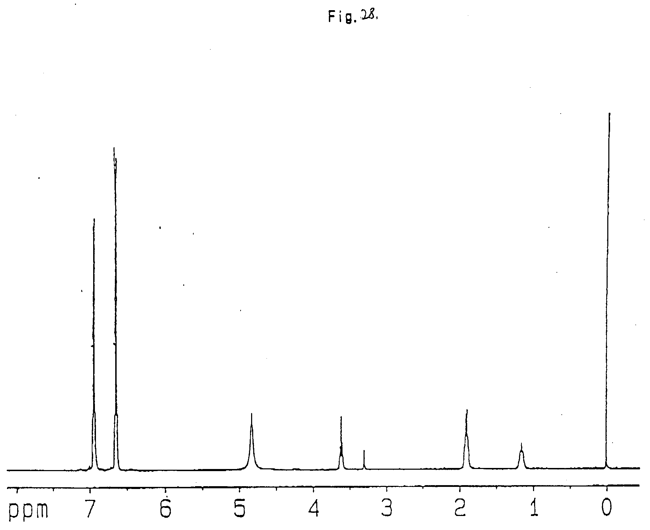

20.0 g of 50 % aqueous solution of glutaric aldehyde and 45.1 g of phenol were applied into 300 ml flask, then 200 ml of the mixture of concentrated sulfuric acid and phosphoric acid (volumetric ratio 3:1) was fed dropwise with stirring over 2 hours at the titration temperature of 0-2°C. Following that, the mixture was stirred for 5 hours at 0 °C. The reaction mixture was poured into 600 ml of ice-water, then the temperature of the solution was raised to 70°C to collect the deposited solid by filtration. The solid was recrystallized from dioxane, then dried under vacuum at 100 °C, affording 11.9 g of whitish powder. The yield was 27.9 %. The powder was identified as 1,1, 5,5-tetrakis(4-hydroxyphenyl)pentane by means of both infrared spectrum and NMR spectrum. The chart of each measurement was shown in FIG. 27 and FIG. 28.

### (1-6) Synthesis of 1,1,4,4-tetrakis(4-hydroxyphenyl)butane:

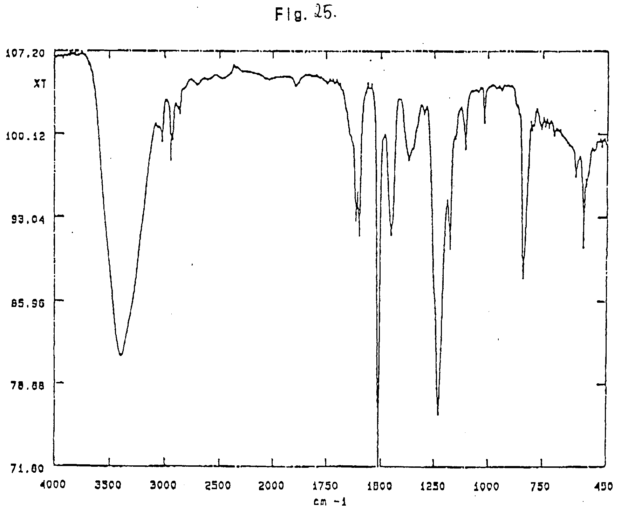

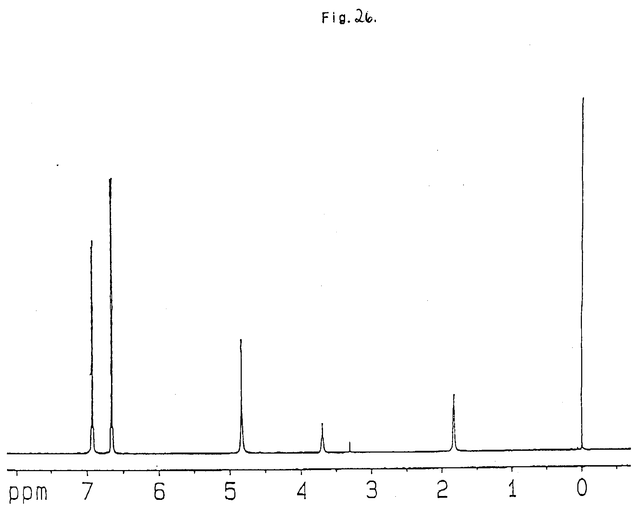

25.0 g of succinaldehyde sodium bisulfite, 38.3 g of phenol and 50 ml of acetic acid were applied into 300 ml flask, then 100 ml of the mixture of concentrated sulfuric acid and phosphoric acid (volumetric ratio 3:1) was fed dropwise with stirring over 2 hours at the titration temperature of 0-2°C. Following that, the solution was stirred for 4 hours at 0°C. The reaction mixture was poured into 600 ml of ice-water, then the temperature of the solution was raised to 70°C to collect the deposited solid by filtration. The solid was recrystallized from dioxane, then dried under vacuum at 100 °C, affording 7.0 g of whitish powder. The yield was 18.1 %. The powder was identified as 1,1,4,4-tetrakis(4-hydroxyphenyl)butane by means of both infrared spectrum and NMR spectrum. The chart of each measurement was shown in FIG. 25 and FIG. 26.

### Example 10

### Sample: Preparation of A-14 through A-49

(1) The clathrate compounds of which guest compound is methanol, ethanol, 2-propanol, acetonitrile, acetone, THF, 1,4-dioxane, benzaldehyde, diethylamine, pyridine or benzene ware prepared by using tetrakisphenol obtained in the synthetic Example 1-1 through 1-6 as host compound. For the preparation of clathrate compounds, 2.5 mmol of host compound was added into 10 ml of guest compound and the mixture was stirred for 1 to 300 minutes in the temperature range from 25 to 100 °C. Then the solution was immediately filtered and the filtrate was allowed to stand at room temperature for 1 to 48 hours to deposit the crystals. The crystals were collected by filtration, then dried under vacuum at room temperature, affording the clathrate compound specified in the present invention.
   The identification of the clathrate compounds was carried out by using TG-DTA (Thermogravimetry-Differential thermal analysis) measurement and infrared spectrum. The manufacturing condition of the clathrate compounds of the Examples of the present application, guest/host mol ratio determined by TG-DTA measurement of the clathrate compounds and the releasing temperature of the guest compounds were summarized in Table 4.
(2) The clathrate compound with guest compound 1,8-cineole was prepared by using 1,1,2,2-tetrakis(3,5-dimethyl-4-hydroxyphenyl)ethane obtained in the Synthesis Example 1-2 as host compound.
   For the preparation of clathrate compounds, 1 mmol of host compound was added into 5 ml of methanol heated to 70 °C and the mixture was stirred until the complete dissolution of the host compound. Then the solution was added to 4 mmol of 1,8-cineole and stirred for 5 minute at 80°C, then immediately filtered. The filtrate was allowed to stand at room temperature for 24 hours to deposit the crystals. The crystals were collected by filtration, then dried under vacuum at room temperature, affording the clathrate compound specified in the present invention. And the identification of the clathrate compound was carried out by means of TG-DTA measurement. The manufacturing condition of the clathrate compound of the present Examples, guest/host mol ratio determined by TG-DTA measurement of the clathrate compound and the releasing temperature of the guest compounds were summarized in Table 5.
(3) The clathrate compound of which guest compound is hinokithiol. thymol, menthol, citral, eugenol, carvone, geraniol, 1,8-cineole, menthone or borneol was prepared by using 1,1,2,2-tetrakis(3-methyl-4-hydroxyphenyl)ethane obtained in the synthetic Example 1-2 as host compound.
   For the preparation of clathrate compounds, 1 mmol of host compound was added into 5 ml ° of methanol heated to 60 C and the mixture was stirred until the complete dissolution of the host compound is made. Then the solution was added to 4 mmol of guest compound and stirred for 5 minute at 60 °C, then immediately filtered. The filtrate was allowed to stand at room temperature for 24 hours to deposit the crystals. The crystals were separated by filtration, then dried under vacuum at room temperature, affording the clathrate compound specified in the present invention. And the identifications of the clathrate compounds were carried out by means of TG-DTA measurement. The manufacturing condition of the clathrate compound of the present Example, guest/host mol ratio determined by TG-DTA measurement of the clathrate compound and the releasing temperature of the guest compounds were summarized in Table 6.

### Example 11

### Preparation of clathrate compounds

1.1555 g (2.85 mmol) of TEP-DF (Asahi Yukizai Industries) was added into 10 ml of water and the mixture was stirred for 30 min. at 25°C to make suspension. Then 20 g (11.4 mmol as CMI) of Kathon WT (Rohm & Haas) was gradually fed dropwise thereto and the mixture stirred for 3 hours to proceed the reaction. Following that, the resulting precipitation was filtered with suction and the filtrate was dried under vacuum at room temperature, affording the sample in pale yellowish powder A-50.

### Example 12

1.1355 g of TEP-DF was added into 10 ml of water and the mixture was stirred for 20 min at 50 °C and dispersed. Then 20 g of Kathon WT was gradually fed dropwise thereto and the mixture was reacted under stirring for 3 hours. Following that, the solution was cooled to room temperature, then the resulting precipitation was filtered with suction and dried under vacuum at room temperature, affording the sample in pale yellowish powder A-51.

### Example 13

1.1355 g (2.85 mmol) of TEP-DF was added into 5 ml of methanol and the mixture was stirred under heating until TEP-DF completely dissolve in the solution. After cooling the solution to 25 °C, 20 g of kathon WT was gradually fed dropwise thereto, then the mixture was allowed to stand at reaction for 1 hours. Following that, the resulting deposit was filtered with suction and the filtrate was dried under vacuum at room temperature, affording the sample in pale yellowish crystals A-52.

### Example 14

1.1355 g (2.85 mmol) of TEP-DF was added into 5 ml of methanol and the mixture was stirred under heating until TEP-DF completely dissolve in the solution. During maintaining the solution at 40°C, 20 g of kathon WT was gradually fed dropwise thereto, then the mixture was allowed to stand at reaction for 15 min. at 40 °C. Following that, the solution was cooled to room temperature to result the deposit. The deposit was filtered with suction and the filtrate was dried under vacuum at room temperature, affording the sample in pale yellowish crystals A-53.

### Comparative Example 1

0.827 g (2.85 mmol) of β-dinaphthol was dissolved in 17 ml of methanol, then 5.04 g (0.368 g as CMI, 2.86 mmol) of kathon WT was added thereto and stirred, thereby dark substance was deposited. The substance was filtered with suction and dried under vacuum, affording the comparative sample C-1.
Note: The analytical value of the water-soluble fungicide (Kathon WT) used in the Example 1 through 4 and Comparative Example 1, is as follows.
- CMI:: 10.1 wt%
- MI :: 3.8 wt%
- Rest:: Magnesium chloride plus Magnesium nitrate plus water

The conditions for preparing the samples of clathrate compounds A-50 through A-53 obtained as described above and the comparative sample of olathrate compound C-1, the included quantity of CMI and MI and the releasing temperature of the guest compounds were shown in Table 7.

The formation of included component was confirmed on each sample of clathrate compound based on the analysis by infrared spectrum, NMR spectrum, X-ray diffraction, DTA, HPLC and TLC. By using X-ray microanalyzer, it was confirmed that all of the clathrate compounds contains neither Magnesium chloride nor Magnesium nitrate.

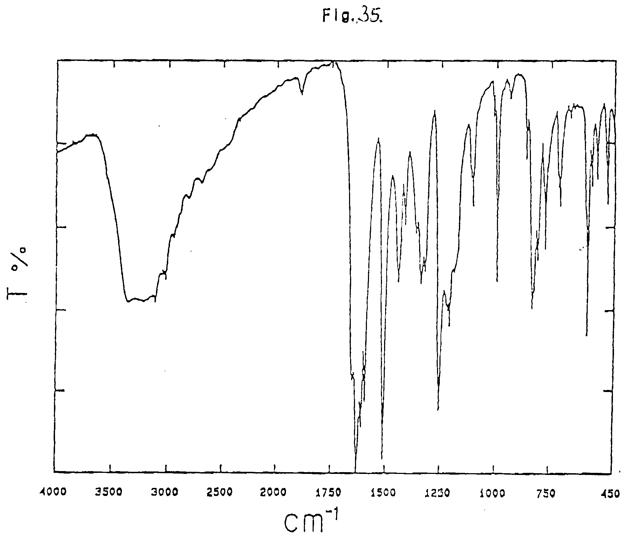

For the release test of CMI, each of the obtained sample A-52, the comparative sample C-1 and CMI was applied into cellulose dialysis membrane at the rate of 10 mg in conversion to CMI, then the membrane was immersed in pure water 11. The periodical change in the amount releasing CMI was checked by determining the released amount of CMI after the prescribed time by using elution tester under stirring at the speed of 100 rpm. The results were summarized in Table 8 and the release curve was shown in FIG. 35.

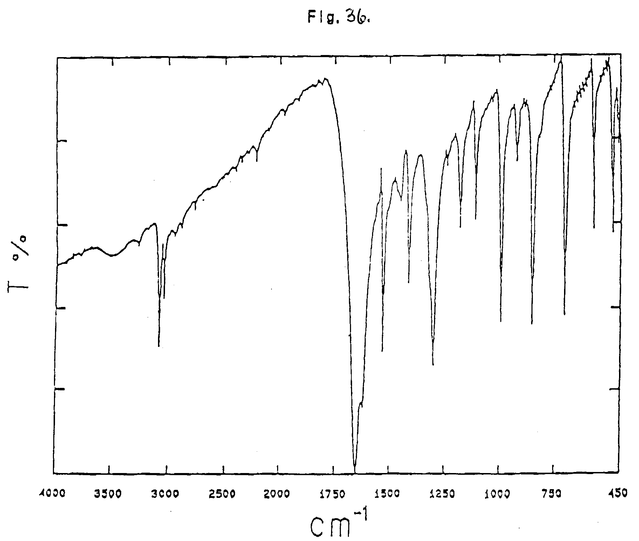

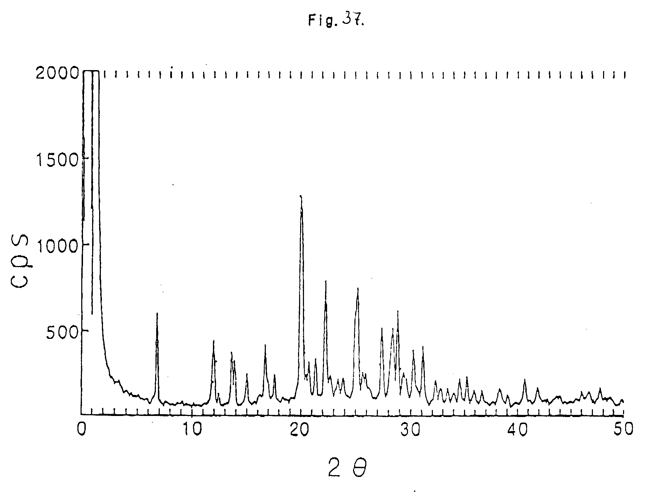

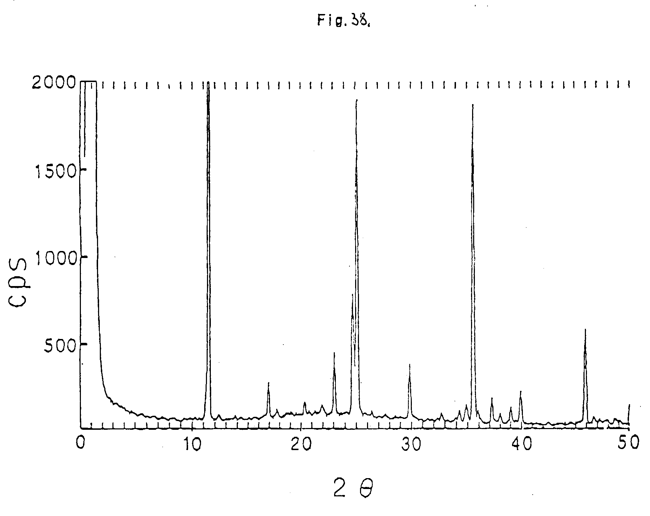

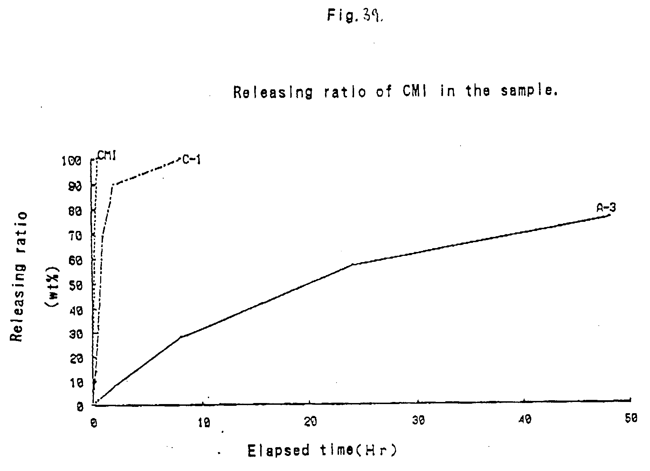

Furthermore, the infrared spectrums of CMI isolated from the sample of the clathrate compound A-52 obtained in this invention and the guest water-soluble disinfectant (Kathon WT) are shown in FIG. 36 and 37 respectively and the diagrams of X-ray diffraction of them are shown in FIG. 38 and 39 respectively.

### INDUSTRIAL APPLICABILITY

The novel clathrate compounds of which the host compound is tetrakisphenol specified in the present invention have the following characteristics.
1) A wide range of organic compounds such as alcohols, ethers, esters, ketones, BTX solvents including benzene and the like, fungicides, heterocyclic compounds containing nitrogen, essential oils, perfumes and the like can be included extremely easily as guest compound.
2) By the inclusion of the compounds having a boiling point near ordinary temperature, the control of volatilization and transpiration can be made.
3) It is possible to selectively recover an organic compound which contaminates water as included component.
4) It is possible to selectively recover a desired compound as included component from a mixture of organic compounds which may not be separated by distillation or other means because of having similar boiling points.
5) It is possible to separate and recover a compound included as guest compound by easily releasing it by heating and the like.
6) When an included component is solid at ordinary temperature, it can be pressed to form tablets and it is very easy for handling.
7) When taking CMI, of which toxicity and skin irritation activity are high and which is a disinfectant for industrial use, as a guest compound and using a compound having lower toxicity such as a tetrakis(hydroxyphenyl)ethane as a host, the toxicity and the skin irritation action can be reduced because CMI is included in a low toxic host compound. Further, since CMI as active ingredient is released over an appropriate period in an aqueous system, it is useful as a disinfectant for a circulatory toilet in a train which is exchanged every 2-3 days. Moreover, as host compound protects CMI as active ingredient, the deterioration of the antimicrobial activity resulting from the reaction of CMI with other substances can be prevented.

As illustrated above, the present invention relates to the clathrate compounds which can include a wide range of organic compounds, and has as an object to provide easy-handling novel clathrate compounds; it also relates to a process for the preparation thereof which is a significant contribution to the industries.

**Table 1**

| Sample No. | Host Compound | Guest Compound | Solvent | Condition for Reaction | | Clathlate Compound | |
|---|---|---|---|---|---|---|---|
| | | | | Temp. (C ° ) | Time (min.) | Guest/Host (mol ratio) | Guest releasing temperature (°C ) |
| A-1 | TEP-DF¹⁾ | pyridine | methanol | 60 | 10 | 3.9 | 111.5 |
| A-2 | " | pyrrole | " | 60 | 10 | 2.0 | 137. |
| A-3 | " | pyrrole | isopropanol | 80 | 30 | 2.2 | 137.7 |
| A-4 | " | pyrazine | methanol | 60 | 20 | 1.3∼1.6 | 76.8 |
| A-5 | " | pyrazole | " | 60 | 20 | 2.9 | 86.3 |
| A-6 | " | imidazole | " | 60 | 20 | 1.3∼1.6 | 103.8 |
| A-7 | " | 1,2,4-triazole | " | 60 | 20 | 1.8∼2.0 | 159.5 |
| A-8 | " | pyridine | - | Room temp. | 3 | 3.9 | 102.9 |
| A-9 | " | pyrrole | - | Room temp. | 5 | 0.9 | 99.9 |

| | | | | | | | |
|---|---|---|---|---|---|---|---|
| Note) ¹⁾:1,1,2,2,-tetrakis(p-hydroxyphenyl)ethane | | | | | | | |

**Table 2**

| Sample No. | Host compound | Guest compound | Solvent | Condition for reaction Clathrate compound | | | |
|---|---|---|---|---|---|---|---|
| | | | | Temp. (°C) | Time (min.) | Guest /Host (mol ratio) | Guest rele asing temp -erature (°C) |
| A-10 | TEP-DF¹⁾ (1.0g) | 1.8-cineole (1.55g) | methanol (5ml) | 60 | 10 | 4.0 | 102.9 |
| A-11 | " | hinokithiol (1.65%) | " | 60 | 10 | 4.0 | 151.6 |
| A-12 | " | 1-menthol (1.57g) | benzene (5ml) | 40 | 30 | 2.5 | 98.6 |
| A-13 | " | α-terpineol (1.55g) | ― | 60 | 5 | 4.0 | 80.5 |
| Note) ¹⁾ :1,1,2,2,-tetrakis(p-hydroxyphenyl)ethane ( ) :Amount used at the reaction. | | | | | | | |

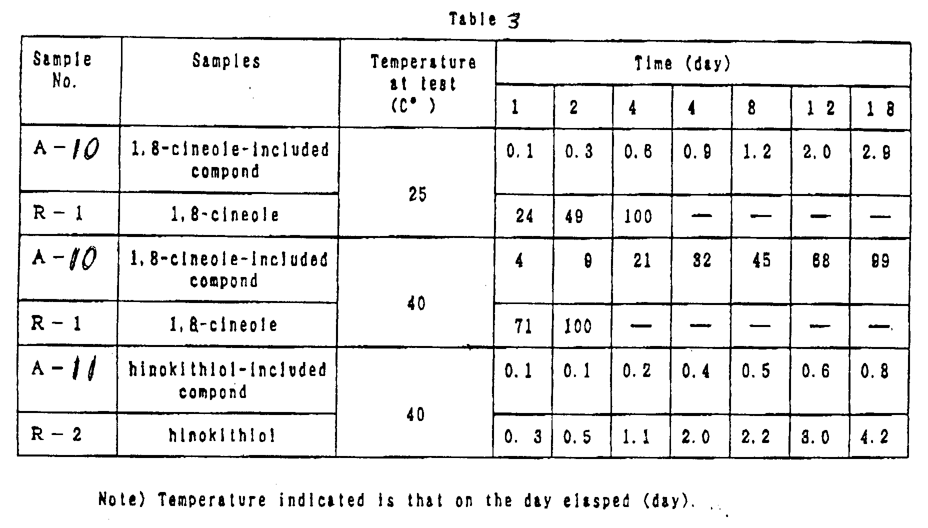

**Table 4**

| Sample No. | Host | Guest compound | Manufacturing condition | | |
|---|---|---|---|---|---|
| | | | Temp. stirred°C | Time stirred min. | Time remained Hr |
| A-14 | 1-1 | 1,4-dioxane | 80 | 5 | 24 |
| A-15 | " | benzaldehyde | 100 | 10 | 24 |
| A-16 | " | benzene | 50 | 10 | 24 |
| A-17 | 1-2 | methanol | 50 | 5 | 24 |
| A-18 | " | ethanol | 60 | 5 | 24 |
| A-19 | " | 2-propanol | 25 | 300 | 1 |
| A-20 | " | acetonitrile | 60 | 5 | 24 |
| A-21 | " | acetone | 50 | 5 | 24 |
| A-22 | " | THF | 50 | 5 | 24 |
| A-23 | " | 1,4-dioxane | 80 | 10 | 24 |
| A-24 | " | diethylamine | 50 | 1 | 24 |
| A-25 | " | triethylamine | 70 | 1 | 24 |
| A-26 | " | pyridine | 80 | 10 | 24 |
| A-27 | " | benzene | 70 | 5 | 21 |
| A-28 | 1-3 | acetonitrile | 60 | 5 | 48 |
| A-29 | " | acetone | 50 | 5 | 24 |
| A-30 | " | THF | 50 | 5 | 24 |
| A-31 | " | pyridine | 80 | 10 | 24 |
| A-32 | 1-4 | 1,4-dioxane | 80 | 10 | 24 |
| A-33 | 1-5 | 2-propanol | 70 | 5 | 48 |
| A-34 | " | acetonitrile | 60 | 5 | 48 |
| A-35 | " | THF | 50 | 5 | 48 |
| A-36 | " | 1,4-dioxane | 80 | 10 | 48 |
| A-37 | 1-6 | THF | 50 | 5 | 48 |
| A-38 | " | 1,4-dioxane | 80 | 10 | 48 |

**Table 5**

| Sample No. | Host | Guest compound | TG-DTA Results | | |
|---|---|---|---|---|---|
| | | | Guest/Host mol ratio | Guest boiling point | Releasing temp. °C |
| A-39 | 1-1 | 1,8-cineole | 2.0 | 170 | 75.8 |

**Table 6**

| Sample No. | Host | Guest compound | TG-DTA Results | | |
|---|---|---|---|---|---|
| | | | Guest/Host mol ratio | Guest boiling point | Releasing temp. °C |
| A-40 | 1-2 | hinokithiol | 2.1 | 141 | 71.1 |
| A-41 | " | thymol | 0.8 | 233 | 78.9 |
| A-42 | " | menthol | 0.6 | 215 | 47.9 |
| A-43 | " | citral | 0.9 | 228 | 111.4 |
| A-44 | " | eugenol | 0.3 | 253 | 70.5 |
| A-45 | " | carvone | 2.0 | 231 | 110.8 |
| A-46 | " | geraniol | 1.0 | 230 | 89.3 |
| A-47 | " | 1,8-cineole | 3.1 | 170 | 63.4 |
| A-48 | " | menthone | 2.0 | 207 | 86.5 |
| A-49 | " | borneol | 1.2 | 214 | 63.0 |

**Table 7**

| Sample No. | Host compound | Guest compound | Solvent for reaction | Reaction Condition | | Guest/Host (mol ratio) | | Guest releasing temperature (°C) |
|---|---|---|---|---|---|---|---|---|
| | | | | Temp °C | Time min | CMI | MI | |
| Example | | | | | | | | |
| A-50 | TEP-DF | Kathon WT | - | 25 | 180 | 1.16 | 0.02 | 152 |
| A-51 | " | " | - | 50 | 30 | 1.21 | 0.02 | 155 |
| A-52 | " | " | methanol | 25 | 60 | 1.96 | 0.02 | 168 |
| A-53 | " | " | " | 40 | 15 | 1.91 | 0.02 | 167 |

| Comparative Example | | | | | | | | |
|---|---|---|---|---|---|---|---|---|
| C-1 | β-dinaphthol | " | " | 25 | 60 | 0.95 | 0.04 | - |

**Table 8**

| Released ratio of CMI in the Sample (Weight%) | | | | | | | |
|---|---|---|---|---|---|---|---|
| | Sample No. | Time elapsed(hour) | | | | | |
| | | 0.25 | 0.5 | 1 | 2 | 24 | 48 |
| Present Invention | A-52 | 1 | 2 | 4 | 8 | 57 | 76 |
| Comparative Example | C-1 | 14 | 33 | 69 | 90 | - | - |
| | CMI | 69 | 100 | - | - | - | - |
| (Note) A-3 : Sample A-3 synthesized in Example 3. C-1 : Sample C-1 synthesized in Comparative Example 1. CMI : 5-chloro-2-methyl-4-isothiazoline-3-one (water-soluble disinfectant, Tradename: Kathon WT, Rohm & Haas Limited). | | | | | | | |

## Claims

1. Inclusion complex comprising a tetrakisphenol derivative represented by the chemical formula (I) as a host compound 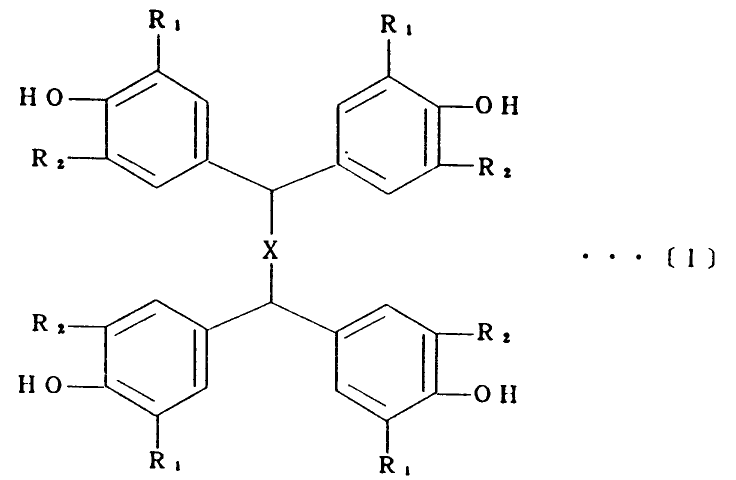 wherein X represents (CH₂)ₙ, n being 1, 2 or 3, and R¹ and R² each independently represent a hydrogen atom, an alkyl group having 1 to 4 carbon atoms, a phenyl group, a halogen atom or an alkoxy group having 1 to 4 carbon atoms;
together with a guest compound.

2. Inclusion complex comprising a tetrakisphenol derivative represented by the chemical formula (I) as a host compound, wherein X represents a direct bond (n being 0), and R¹ represents an alkyl group having 1 to 4 carbon atoms, a phenyl group, a halogen atom or an alkoxy group having 1 to 4 carbon atoms, and R² represents a hydrogen atom or a group represented by R¹;
together with a guest compound.

3. Inclusion complex according to claim 1 or 2, wherein said guest compound is a monocyclic nitrogen-containing heterocycle or a natural essential oil or synthetic perfume selected from 1,8-cineole, hinokithiol, menthol, terpineol, borneol, nopol, citral, citronellol, citronellal, geraniol, linalool, menthone, thymol, carvone, eugenol, dimethyloctanol, fragrant orange-coloured olive, jasmine, and lemon oil.

4. Inclusion complex comprising a tetrakisphenol derivative represented by the chemical formula (I) as host compound, wherein X represents a direct bond (n being 0), and R¹ and R² represent a hydrogen atom;
together with a monocyclic nitrogen-containing heterocycle or a natural essential oil or synthetic perfume selected from 1,8-cineole, hinokithiol, menthol, terpineol, borneol, nopol, citral, citronellol, citronellal, geraniol, linalool, menthone, thymol, carvone, eugenol, dimethyloctanol, fragrant orange-coloured olive, jasmine, and lemon oil, as a guest compound.

## Patentansprüche

1. Einschlußkomplex, umfassend ein Tetrakisphenolderivat, angegeben durch die chemische Formel (I) als Wirtsverbindung 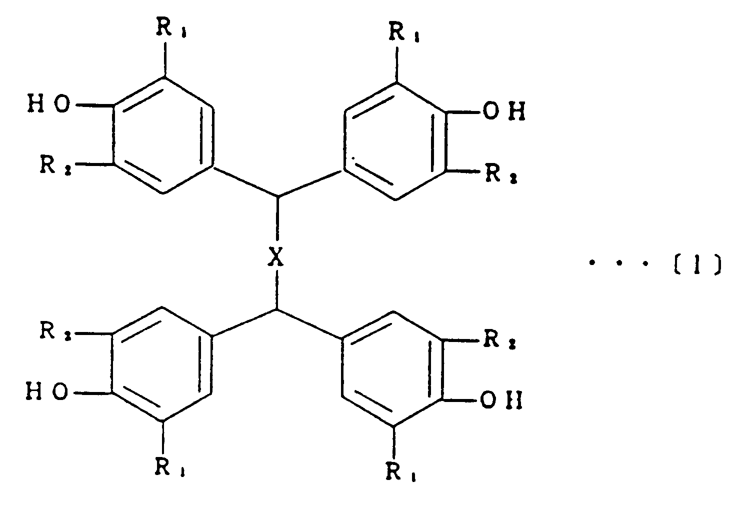 wobei X (CH₂)ₙ bedeutet, n 1, 2 oder 3 ist und R¹ und R² jeweils unabhängig ein Wasserstoffatom, eine Alkylgruppe mit 1 bis 4 Kohlenstoffatomen, eine Phenylgruppe, ein Halogenatom oder eine Alkoxygruppe mit 1 bis 4 Kohlenstoffen bedeuten, zusammen mit einer Gastverbindung.

2. Einschlußkomplex, umfassend ein Tetrakisphenolderivat, angegeben durch die chemische Formel (I) als Wirtsverbindung, wobei X eine direkte Bindung (n = 0) bedeutet und R¹ eine Alkylgruppe mit 1 bis 4 Kohlenstoffatomen, eine Phenylgruppe, ein Halogenatom oder eine Alkoxygruppe mit 1 bis 4 Kohlenstoffatomen bedeutet und R² ein Wasserstoffatom oder eine Gruppe, angegeben durch R¹, bedeutet,
zusammen mit einer Gastverbindung.

3. Einschlußkomplex nach Anspruch 1 oder 2, wobei die Gastverbindung ein monocyclischer stickstoffhaltiger Heterocyclus oder ein natürliches essentielles Öl oder synthetisches Parfüm ist, ausgewählt aus 1,8-Cineol, Hinokithiol, Menthol, Terpinol, Borneol, Nopol, Citral, Citronellol, Citronellal, Geraniol, Linalool, Menthon, Thymol, Carvon, Eugenol, Dimethyloctanol, orange-farbenen Duftoliven, Jasmin und Lemonenöl.

4. Einschlußkomplex, umfassend ein Tetrakisphenolderivat, angegeben durch die chemische Formel (I) als Wirtsverbindung, wobei X eine direkte Bindung (n = 0) ist und R¹ und R² ein Wasserstoffatom bedeuten,
zusammen mit einem monocyclischen stickstoffhaltigen Heterocyclus oder einem essentiellen Öl oder synthetischem Parfüm, ausgewählt aus 1,8-Cineol, Hinokithiol, Menthol, Terpinol, Borneol, Nopol, Citral, Citronellol, Citronellal, Geraniol, Linalool, Menthon, Thymol, Carvon, Eugenol, Dimethyloctanol, orange-farbenen Duftoliven, Jasmin und Lemonenöl als Gastverbindung.

## Revendications

1. Complexe d'insertion comprenant un dérivé de tétrakisphénol représenté par la formule chimique (I) en tant que composé hôte 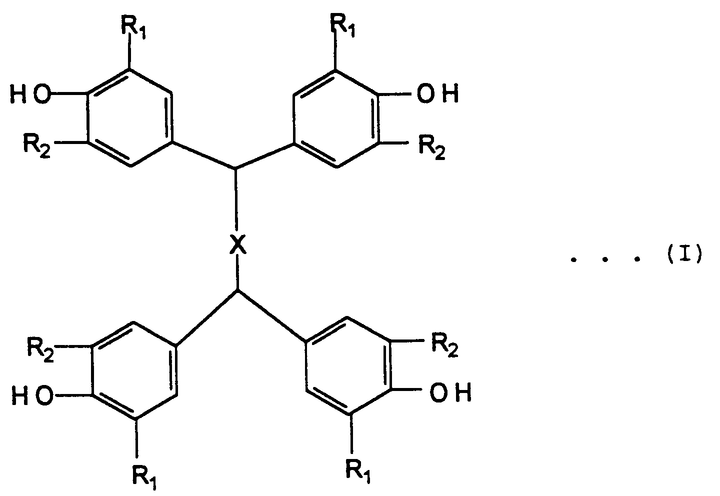 où X représente (CH₂)ₙ, n étant 1, 2 ou 3, et chacun de R¹ et R² représente indépendamment un atome d'hydrogène, un groupe alkyle ayant 1 à 4 atomes de carbone, un groupe phényle, un atome d'halogène ou un groupe alcoxy ayant 1 à 4 atomes de carbone;
avec un composé invité.

2. Complexe d'insertion comprenant un dérivé de tétrakisphénol représenté par la formule chimique (I) en tant que composé hôte, où X représente une liaison directe (n étant 0), et R¹ représente un groupe alkyle ayant 1 à 4 atomes de carbone, un groupe phényle, un atome d'halogène ou un groupe alcoxy ayant 1 à 4 atomes de carbone, et R² représente un atome d'hydrogène ou un groupe représenté par R¹;
avec un composé invité.

3. Complexe d'insertion selon la revendication 1 ou 2, où ledit composé invité est un hétérocycle contenant de l'azote monocyclique ou bien une huile essentielle naturelle ou un parfum synthétique sélectionné parmi le 1,8-cinéole, l'hinokithiol, le menthol, le terpinéol, le bornéol, le nopol, le citral, le citronellol, le citronellal, le géraniol, le linalool, la menthone, la thymol, la carvone, l'eugénol, le diméthyloctanol, l'olive de couleur orange parfumée, le jasmin, et l'essence de citron.

4. Complexe d'insertion comprenant un dérivé de tétrakisphénol représenté par la formule chimique (I) en tant que composé hôte, où
X représente une liaison directe (n étant 0), et R¹ et R² représentent un atome d'hydrogène;
avec un hétérocycle contenant de l'azote monocyclique ou une huile essentielle naturelle ou un parfum synthétique sélectionné parmi le 1,8-cinéol, l'hinokithiol, le menthol, le terpinéol, le bornéol, le nopol, le citral, le citronellol, le citronellal, le géraniol, le linalool, la menthone, le thymol, la carvone, l'eugénol, le diméthyloctanol, l'olive de couleur orange parfumée, le jasmin, et l'essence de citron, en tant que composés invités.
